Europäisches Patentamt

European Patent Office

Office européen des brevets

(11) Veröffentlichungsnummer : **0 086 476**
**B1**

(12) **EUROPÄISCHE PATENTSCHRIFT**

(45) Veröffentlichungstag der Patentschrift :
**07.05.86**

(51) Int. Cl.⁴ : **A 61 K 31/59**

(21) Anmeldenummer : **83101341.2**

(22) Anmeldetag : **11.02.83**

(54) Verwendung von Cholecalciferolderivaten.

(30) Priorität : **12.02.82 US 348389**

(43) Veröffentlichungstag der Anmeldung :
**24.08.83 Patentblatt 83/34**

(45) Bekanntmachung des Hinweises auf die Patenterteilung : **07.05.86 Patentblatt 86/19**

(84) Benannte Vertragsstaaten :
**BE CH DE FR GB IT LI LU NL SE**

(56) Entgegenhaltungen :
**GB-A- 2 060 642**

(73) Patentinhaber : **F. HOFFMANN-LA ROCHE & CO.
Aktiengesellschaft
CH-4002 Basel (CH)**

(72) Erfinder : **Boris, Alfred
25 Lord Sterling Drive
Parsippany, N.J. (US)**
Erfinder : **Partridge, John Joseph
121 Norwood Avenue
Upper Montclair, N.J. (US)**
Erfinder : **Uskokovic, Milan Radoje
253 Highland Avenue
Upper Montclair, N.J. (US)**

(74) Vertreter : **Lederer, Franz, Dr. et al
Patentanwälte Dr. Franz Lederer Dipl.-Ing. Reiner F.
Meyer-Roxlau Lucile-Grahn-Strasse 22
D-8000 München 80 (DE)**

**Beschreibung**

Aus GB-A-2060642 ist die Verwendung des 1α,25 (R,S), 26-Trihydroxycholecalciferols als Wirkstoff zur Regulierung des Calciumhaushaltes bzw. Calciumtransportes im Körper bekannt.

Die Erfindung beruht auf dem Auffinden von neuen physiologischen Eigenschaften der C-25R- und S-Epimeren des 1α,25,26-Trihydroxycholecalciferols, die weiter unten das R- und das S-Epimere genannt werden. Insbesondere wurde gefunden, dass das R- und das S-Epimere die über dem Normalwert liegenden Spiegel von endogen produziertem 1α,25-Dihydroxycholecalciferol senken.

Dementsprechend betrifft die Erfindung das R- oder S-Epimere als Wirkstoff zur Behandlung von Krankheiten, die durch über dem Normalwert liegende Spiegel von endogen produziertem 1α,25-Dihydroxycholecalciferol im Serum oder durch erhöhte Empfindlichkeit auf 1α,25-Dihydroxycholecalciferol gekennzeichnet sind. Die Erfindung betrifft ferner die Verwendung des R- oder S-Epimeren zur Herstellung von pharmazeutischen Präparaten für die obige Behandlung, sowie pharmazeutische Präparate auf des Basis des R- oder S-Epimeren für diese Behandlung.

Als Beispiele der obigen Krankheiten sind Hypercalcämie, Sarcoidosis, Hypercalciurie, Nephrolithiasis und Nephrocalcinosis zu nennen.

Wie oben angegeben, senken das R- und das S-Epimere den 1α,25-Dihydroxycholecalciferol-Spiegel im Serum. Ferner bewirkt das R- und das S-Epimere die Mineralisierung der Knochen bei Vitamin D-armen Tieren, aber nur das R-Epimere bewirkt die Mineralisierung der Knochen bei mit Dinatriumäthan-1-hydroxy-1,1-diphosphonat blockierten Tieren.

Die obigen Aktivitäten können in den folgenden Versuchen nachgewiesen werden :

a) Antirachitische Aktivität

Weissen Leghorn-Küken wird unter UV-freier Beleuchtung eine Vitamin D-arme, 1 % Calcium und 0,7 % Phosphor enthaltende Diät gegeben. Die in Propylenglykol gelösten Testverbindungen werden während 21 Tagen den am Anfang des versuchs 1 oder 2 Tage alten Küken oral verabreicht. Kontrolltiere werden mit dem Vehikel behandelt. Die Küken werden am Tag nach dem letzten Behandlungstag seziert und das Tibiaaschengewicht wird ermittelt. Es werden 9 oder 10 Küken für jede Behandlungsgruppe und für die Kontrollgruppe verwendet. Die mittleren Tibiaaschengewichte ausgedrückt in mg sind in der nachfolgenden Tabelle I angegeben. Die Resultate zeigen, dass das R- und das S-Epimere eine beträchtliche antirachitische Aktivität aufweisen.

Tabelle I

| Dosis (mg/Küken/Tag) | Mittleres Tibiaaschengewicht (mg) | |
|---|---|---|
| | S-Epimere | R-Epimere |
| 0 | 112,1 ± 6,2 | |
| 30 | 124,9 ± 2,8 | 122,6 ± 4,1 |
| 100 | 159,4 ± 4,4 | 157,5 ± 5,3 |
| 300 | 187,0 ± 8,7 | 207,8 ± 7,8 |
| 1 000 | 210,4 ± 7,8 | 213,3 ± 10,0 |

b) Intestinale Calcium-Absorption bei Küken

Weissen Leghorn-Küken wird während 21 Tagen unter UV-freier Bestrahlung eine Vitamin D-arme Diät gegeben. Eine Einzeldosis der Testverbindung, gelöst in Propylenglykol, wird den Tieren oral verabreicht. Danach werden zu verschiedenen Zeitpunkten $7,410^{10}$ s$^{-1}$ (2 µCi) $^{45}$Ca (Chlorid) oral verabreicht und die Serumradioaktivität 45 Minuten nach Verabreichung des Isotops gemessen. In jeder Versuchsperiode werden Kontrolltiere verwendet, denen nur das Vehikel verabreicht wird. In jeder Behandlungsgruppe und jeder Kontrollgruppe werden 10 Küken verwendet. Die in der Tabelle II gegebenen Resultate zeigen, dass das S- und das R-Epimere ähnliche intestinale $^{45}$Ca-Absorption bewirken.

**0 086 476**

Tabelle II

| Behandlung | Zeit | Serum $^{45}$Ca (cpm/0,2 ml) |
|---|---|---|
| | (Stunden) | |
| Vehikel, 0,2 ml | 3 | 1 338 ± 51 |
| S-Epimere | | 1 977 ± 122 |
| R-Epimere | | 2 077 ± 174 |
| Vehikel, 0,2 ml | 6 | 1 345 ± 89 |
| S-Epimere | | 2 067 ± 128 |
| R-Epimere | | 1 992 ± 212 |

c) Verhütung der durch EHDP-induzierten Blockierung der Mineralisierung bei Ratten

Männliche Charles River Ratten werden 10 Tage mit Dinatrium-äthan-1-hydroxy-1,1-diphosphonat (EHDP) behandelt. Die Verbindung wird in destilliertem Wasser in einer Konzentration von 2 mg/0,2 ml/Ratte jeden Tag subcutan verabreicht. Die Testverbindungen werden in Propylenglykol jeden Tag oral verabreicht. Am Tag nach der letzten Behandlung werden die Ratten seziert und deren Tibia durch Silberimprägnierung der Knochensalze behandelt. Die Breite der Epiphysenplatte wird mit einem Mikroskop gemessen. Zur Ermittlung der Aktivität wird die dosisabhängige Verengerung der durch EHDP-Induktion erweiterten Epiphysenplatte gemessen. In jeder Behandlungsgruppe werden 10 Ratten verwendet. Es werden auch positive (EHDP allein) und negative (Vehikel allein) Kontrollgruppen von 10 Ratten in jedem Versuch verwendet. Die in der Tabelle III angegebenen Resultate zeigen, dass im Gegensatz zum S-Epimeren das R-Epimere die Calcifizierung der Tibiaepiphysenplatte bei EHDP-blockierten Ratten bewirkt.

Tabelle III

| Dosis (µg/Ratte/Tag) | Mittlere Breite der Epiphysenplatte (µm) | | |
|---|---|---|---|
| | EHDP + S-Epimere | EHDP allein | EHDP + R-Epimere |
| 0 | | 1 190 ± 42 | |
| 10 | 1 162 ± 36 | | 611 ± 51 |

Vehikel-Kontrollen (kein EHDP) 376 ± 13

d) Einfluss auf die $1\alpha,25\,(OH)_2D_3$- und $25\,(OH)D_3$-Spiegel im Serum

Der Einfluss der subcutanen Verabreichung der S- und R-Epimeren auf die $1\alpha,25$-Dihydroxycholecalciferol- und 25-Hydroxycholecalciferol ($1\alpha,25\,(OH)_2D_3$ und $25(OH)D_3$)-Spiegel wurde nach bekannten Methoden (Archives of Biochemistry and Biophysics, Band 201, No. 1, 1980, 277-285) ermittelt. Die Resultate sind in der Tabelle IV wiedergegeben. Der Einfluss wurde in einem 7 Tage-Versuch und dann in einem 28 Tage-Versuch ermittelt. Es hat sich dabei erwiesen, dass die Senkung des $1\alpha,25\,(OH)_2D_3$-Spiegels anhält. Die Fähigkeit, die endogene Produktion von $1\alpha,25\,(OH)_2D_3$ zu kontrollieren, ist bei der Behandlung von Krankheiten, wie Sarcoidosis und Hypercalciurie anwendbar.

Tabelle IV

| Tägliche Behandlung | Anzahl Tage | Anzahl Ratten | Serumspiegel | |
|---|---|---|---|---|
| | | | $1\alpha,25(OH)_2D_3$ (pg/ml) | $25(OH)D_3$ (ng/ml) |
| Vehikel 0,2 ml | 7 | 18 | 99,6 ± 0,5 | 37,6 ± 1,9 |
| S-Epimere 1 mcg | | 12 | 7,9 ± 2,9 | 32,0 ± 1,7 |
| R-Epimere 1 mcg | | 12 | 14,0 ± 4,1 | 36,0 ± 1,8 |
| Vehikel 0,2 ml | 28 | 10 | 32,8 ± 7,3 | 64,2 ± 4,3 |
| S-Epimere 1 mcg | | 10 | 8,0 ± 2,3 | 40,6 ± 3,4 |
| R-Epimere 1 mcg | | 10 | 8,2 ± 1,7 | 34,7 ± 4,2 |

3

Das R- und das S-Epimere können in Tagesdosen im Bereich von 0,5 bis 500 µg verabreicht werden. Sie werden vorzugsweise oral, z. B. in Form von Tabletten, Kapseln oder Elixieren verabreicht, können aber auch parenteral, insbesondere subcutan, intramuskulär, intravenös oder intraperitoneal, z. B. in Form von sterilen Lösungen oder Suspensionen, verabreicht werden. Etwa 0,5 bis 500 µg des R- oder des S-Epimeren werden mit pharmazeutisch anwendbaren Hilfsstoffen, wie Vehikeln, Trägern, Excipientien, Bindemitteln, Konservierungsmitteln, Stabilisierungsmitteln und/oder Geschmackstoffen, in einer Einzeldosis vermischt werden.

Beispiele von Hilfsmittel, die in Tabletten oder Kapseln verwendet werden können, sind Bindemittel, wie Maisstärke oder Gelatine ; Excipientien, wie Dicalciumphosphat ; Zersetzungsmittel, wie Mais- oder Kartoffelstärke oder Alginsäure ; Gleitmittel, wie Magnesiumstearat ; Süssstoffe, wie Sucrose ; Geschmacksmittel, wie Pfefferminze. Es können andere Materialien, wie Ueberzüge zur Modifizierung des Aspektes der Einzeldosen, verwendet werden. Tabletten können z. B. mit Shellack, Zucker oder beide überzogen werden. Ein Sirup oder Elixier kann die Aktivsubstanz, Sucrose als Süssmittel, Methyl- und Propylparaben als Konservierungsmittel, ein Farbstoff und einen Geschmackstoff, wie Orangearoma, enthalten.

Sterile Präparate zum Injizieren können in üblicher Weise dadurch hergestellt werden, dass man das R- oder das S-Epimere in einem Vehikel, wie Injektionswasser, einem in der Natur vorkommenden pflanzlichen Oel, wie Sesamöl, oder einem synthetischen fetten Bindemittel, wie Aethyloleat, auflöst oder suspendiert. Es können auch Puffer, Konservierungsmittel und Antioxidantien zugesetzt werden.

## Beispiel 1

Die Bestandteile 1 bis 4 werden gemischt und nötigenfalls gemahlen. Nach Zusatz des Magnesiumstearats wird das Gemisch gemahlen und zu Tabletten gepresst.

|  | mg/Tablette | | |
|---|---|---|---|
| 1. Das R- oder S-Epimere des 1α,25,26-Trihydroxycholecalciferols | 0,025 | 0,100 | 0,5 |
| 2. Lactose | 157,975 | 157,900 | 157,5 |
| 3. Mikrokristalline Cellulose | 20,000 | 20,000 | 20,0 |
| 4. Modifizierte Stärke | 20,000 | 20,000 | 20,0 |
| 5. Magnesiumstearat | 2,000 | 2,000 | 2,0 |
| Gesamtgewicht | 200,000 | 200,000 | 200,0 |

## Beispiel 2

Die Bestandteile 2 und 3 werden vermischt und mit einer Lösung des Bestandteils 1 in Alkohol besprüht und dann über Nacht getrocknet. Das Gemisch wird gesiebt, dann mit Talk vermischt und in Kapseln gefüllt.

|  | mg/Kapsel | | |
|---|---|---|---|
| 1. Das R- oder S-Epimere des 1α,25,26-Trihydroxycholecalciferols | 0,025 | 0,100 | 0,500 |
| 2. Lactose | 159,975 | 159,90 | 159,50 |
| 3. Modifizierte Stärke | 20,0 | 20,0 | 20,0 |
| 4. Talk | 20,0 | 20,0 | 20,0 |
| Gesamtgewicht | 200 | 200 | 200 |

## Beispiel 3

Das R- oder S-Epimere des 1α,25,26-Trihydroxycholecalciferols kann in einem pharmazeutisch verwendbaren Lösungsmittel wie Alkohol, Propylenglykol, Glycerin oder Polyäthylenglykol aufgelöst werden. Oberflächenaktive Mittel, wie Polyäthylenglykol, Sorbitanester, Dioctylnatriumsulfosuccinate, Polyoxyäthylen-polyoxypropylen-Copolymere, können zur Solubilisierung zugesetzt werden. Ein Konservierungsmittel zur Verhütung des mikrobiellen Wachstums kann auch zugesetzt werden. Beispiele von aus solchen Gemischen zusammensetzbaren Kapselformulierungen sind :

| a) | mg/Kapsel | | |
|---|---|---|---|
| Das R- oder S-Epimere des 1α,25,26-Trihydroxycholecalciferols | 0,025 | 0,1 | 0,50 |
| Polyäthylenglykol (PEG) | 400,0 | 400,0 | 400,00 |
| Butyliertes Hydroxyanisol (BHA) | 0,2 | 0,2 | 0,2 |
| Ascorbylpalmitat | 1,0 | 1,0 | 1,0 |

4

Das R- oder S-Epimere des 1α,25,26-Trihydroxycholecalciferols wird einer Lösung von BHA und Ascorbylpalmitat in PEG zugesetzt und das Gemisch wird unter einer Stickstoffatmosphäre aufgelöst. Die Flüssigkeit wird in Weichkapseln abgefüllt.

b)

| | mg/Kapsel | | |
|---|---|---|---|
| Das R- oder S-Epimere des 1α,25,26-Trihydroxy-cholecalciferols | 0,025 | 0,1 | 0,50 |
| PEG 400 (oder PEG 6000) | 200,0 | 200,0 | 200,0 |
| Polyoxyäthylensorbitanmonooleat oder -monostearat (Polysorbat 80 oder Polysorbat 60) | 200,0 | 200,0 | 200,0 |
| BHA | 0,2 | 0,2 | 0,2 |
| Ascorbylpalmitat | 1,0 | 1,0 | 1,0 |

Das Gemisch von PEG 6 000 und Polysorbat 60 wird erhitzt. Das BHA und das Ascorbylpalmitat werden zugesetzt. Dann wird das R- oder das S-Epimere des 1α,25,26-Trihydroxycholecalciferols unter einer Stickstoffatmosphäre zugesetzt. Das Gemisch wird in Hartkapseln abgefüllt.

c)

| | mg/Kapsel | | |
|---|---|---|---|
| Das R- oder S-Epimere des 1α,25,26-Trihydroxy-cholecalciferols | 0,025 | 0,1 | 0,50 |
| PEG 400 | 100,0 | 100,0 | 100,0 |
| PEG 4000 | 300,0 | 300,0 | 300,0 |
| BHA | 0,1 | 0,1 | 0,1 |
| Butyliertes Hydroxytoluol (BHT) | 0,1 | 0,1 | 0,1 |
| Ascorbylpalmitat | 1,0 | 1,0 | 1,0 |

Das Gemisch von PEG 400 und PEG 4 000 wird erhitzt. BHT, BHA und das Ascorbylpalmitat werden zugesetzt. Dann wird das R- oder S-Epimere des 1α,25,26-Trihydroxycholecalciferols zugesetzt und unter einer Stickstoffatmosphäre gelöst. Das Gemisch wird in Hartkapseln abgefüllt.

**Patentansprüche**

1. Das C-25 R- oder S-Epimere des 1α,25,26-Trihydroxycholecalciferols als Wirkstoff zur Behandlung von Krankheiten, die durch über dem Normalwert liegende Spiegel von endogen produziertem 1α,25-Dihydroxycholecalciferol im Serum oder durch erhöhte Empfindlichkeit auf 1α,25-Dihydroxycholecalciferol gekennzeichnet sind.

2. Die Verwendung des C-25 R- oder S-Epimeren des 1α,25,26-Trihydroxycholecalciferols zur Herstellung von pharmazeutischen Präparaten für die Behandlung von Krankheiten, die durch über dem Normalwert liegende Spiegel von endogen produziertem 1α,25-Dihydroxycholecalciferol im Serum oder durch erhöhte Empfindlichkeit auf 1α,25-Dihydroxycholecalciferol gekennzeichnet sind.

3. Verwendung des C-25 R- und S-Epimeren des 1α,25,26-Trihydroxycholecalciferols zur Herstellung von pharmazeutischen Präparaten zur Behandlung von Hypercalcämie, Sarcoidosis, Hypercalciurie, Nephrolithiasis oder Nephrocalcinosis.

4. Pharmazeutische Präparate enthaltend das C-25 R- oder S-Epimere des 1α,25,26-Trihydroxycholecalciferols in Kombination mit einem pharmazeutisch verwendbaren inerten Trägermaterial zur Behandlung nach dem Anspruch 2 oder 3.

5. Pharmazeutisches Präparat gemäss Anspruch 4, enthaltend 0,5 bis 500 μg des C-25 R- oder S-Epimeren des 1α,25,26-Trihydroxycholecalciferols.

**Claims**

1. The C-25 R- or S-epimer of 1α,25,26-trihydroxycholecalciferol as an active substance for the treatment of disease states, which are characterized by a level of endogenously-produced 1α,25-dihydroxycholecalciferol in the serum which lies above the normal value or by an increased sensitivity to 1α,25-dihydroxycholecalciferol.

2. The use of the C-25 R- or S-epimer of 1α,25,26-trihydrocholecalciferol for the manufacture of pharmaceutical preparations for the treatment of disease states, which are characterized by a level of endogenously-produced 1α,25-dihydroxycholecalciferol in the serum which lies above the normal value or by an increased sensitivity to 1α,25-dihydroxycholecalciferol.

3. Use of the C-25 R- and S-epimer of 1α,25,26-trihydroxycholecalciferol for the manufacture of pharmaceutical preparations for the treatment of hypercalcemia, sarcoidosis, hypercalciuria, nephrolithiasis or nephrocalcinosis.

**0 086 476**

4. Pharmaceutical preparations containing the C-25 R- or S-epimer of 1α,25,26-trihydroxycholecalciferol in combination with a pharmaceutically usable inert carrier material for the treatment according to claim 2 or 3.

5. A pharmaceutical preparation in accordance with claim 4, containing 0.5 to 500 μg of the C-25 R- or S-epimer of 1α,25,26-trihydroxycholecalciferol.

**Revendications**

1. L'épimère R ou S en C-25 du 1 alpha,25,26-trihydroxycholécalciférol en tant que substance active pour le traitement de maladies qui se caractérisent par un niveau dans le sérum de 1 alpha,25-dihydroxycholécalciférol de formation endogène supérieur à la valeur normale ou par une sensibilité accrue au 1 alpha,25-dihydroxycholécalciférol.

2. L'utilisation de l'épimère R ou S en C-25 du 1 alpha,25,26-trihydroxycholécalciférol pour la préparation de compositions pharmaceutiques destinées au traitement de maladies qui se caractérisent par un niveau dans le sérum de 1 alpha,25-dihydroxycholécalciférol de formation endogène supérieur à la valeur normale ou par une sensibilité accrue au 1 alpha,25-dihydroxycholécalciférol.

3. L'utilisation de l'épimère R et S en C-25 du 1 alpha,25,26-trihydroxycholécalciférol pour la préparation de compositions pharmaceutiques destinées au traitement de l'hypercalcémie, de la sarcoïdose, de l'hypercalciurie, de la néphrolithiase ou de la néphrocalcinose.

4. Compositions pharmaceutiques contenant l'épimère R ou S en C-25 du 1 alpha,25,26-trihydroxycholécalciférol en combinaison avec un véhicule inerte acceptable pour l'usage pharmaceutique pour le traitement selon la revendication 2 ou 3.

5. Composition pharmaceutique selon la revendication 4, contenant de 0,5 à 500 microgrammes de l'épimère R ou S en C-25 du 1 alpha,25,26-trihydroxycholécalciférol.

6